# EUROPEAN PATENT APPLICATION

(11) **EP 3 881 870 A1**
(43) Date of publication of application: **22.09.2021**
(21) Application number: 20382212.7
(22) Date of filing: 20.03.2020
(51) Int. Cl.: A61L 9/01

(54) **METHODS AND SYSTEMS FOR DISPENSING A CUSTOMIZED FRAGRANCE**

(71) Applicant: Noustique Perfumes, S.L., 08002 Barcelona (ES)
(72) Inventor: Lasala Alonso, Hugo, 50006 ZARAGOZA (ES); Suarez Iribarne, Alvaro, 08008 BARCELONA (ES)
(74) Representative: ZBM Patents - Zea, Barlocci & Markvardsen

(57) **Abstract**

A method for dispensing a customized fragrance by a fragrance dispensing apparatus is provided. The method comprises providing a plurality of cartridges comprising one or more scents, wherein each cartridge is associated with a respective audio file of a plurality of audio files, and wherein each audio file comprises a plurality of encoded blending parameters. The method also comprises receiving, via a user interface, a customized audio file and decoding the customized audio file to obtain a set of customized blending parameters. The method further comprises dispensing a customized fragrance composition based on the customized blending parameters. A computer program and systems for dispensing a customized fragrance are also provided.

## Description

The present disclosure relates to methods for dispensing a customized fragrance, and to computer programs and systems suitable for performing such methods.

### BACKGROUND

Dispensing devices for dispensing fluids e.g. perfume, hair dye, etc., are known. Such solutions may involve a plurality of fluid containers or cartridges comprising a predetermined amount one or more scents from which the contents are extracted and dispensed.

Known devices, usually comprise a user-driven selection tools for creating a customized fragrance blend. Typically, such selection tools comprise a plurality of options which the user may select in order to create a customized fragrance. Such selection tools, however, comprise some drawbacks which render the fragrance creating process complex and difficult.

Current user-driven selection tools are normally difficult to be used by average users. Mainly because current selection tools do not offer any clue of the final blend nor the effects of possible changes in the selected composition e.g. when the amount of a selected scent is reduced or when a further scent is selected.

Another drawback of said prior art systems is that by mere selection of a plurality of different fragrances it is very difficult to obtain a technically correct or balanced fragrance blend without the expertise level of a professional perfumier.

In addition, an absence of a ubiquitous and/or accessible record register, e.g. a memory, may make difficult to remember the exact composition of a specific fragrance blend. An easy reproduction of formerly created fragrance blends may thus be hardly implemented.

In conclusion, it would be desirable to provide methods aimed at improving current user-driven selection tools for defining fragrance compositions which are more user friendly and can provide technically correct fragrance blends.

### SUMMARY

In a first aspect a method for dispensing a customized fragrance by a fragrance dispensing apparatus is provided. The method comprises providing a plurality of cartridges comprising one or more scents, wherein each cartridge is associated with respective audio files of a plurality of audio files, and wherein each audio file comprises a plurality of blending parameters. The method also comprises receiving, via a user interface, a customized audio file and decoding the customized audio file to obtain a set of customized blending parameters. The method further dispensing a customized fragrance composition based on the customized blending parameters.

By using audio files associated to a respective cartridge, and thus to the one or more scents comprised therein, the average user may not require knowledge about fragrances and/or perfumery which renders the dispensing of customized fragrance blends more user friendly.

In addition, an audio is easier to remember and to identify than either a smelled scent or the plurality of components and their proportions, and thus, the use of audio files facilitate remembering past customized fragrance blends. The reproduction of a previously dispensed fragrances is thus facilitated.

Moreover, the use of audio files may avoid dispensing unsatisfactory blends and the need to perform several tests to reproduce a formerly dispensed fragrance which prevents wasting the content of the cartridges thereby substantially reduces the costs.

In order to clarify some of the terms used throughout the present description and claims some definitions will be given.

The term "customized fragrance" is to be understood as scent blend created by the user.

The term "scent" is to be understood as a blend of natural and or synthetic ingredients, e.g. a blend of notes such as lemon, grass and orange. The "content" of a cartridge and "scent" may be interchangeably used herein.

In the present specification, a "blending parameter" may be understood as a parameter comprising information about e.g. the composition, the amount of scent to be released from a particular cartridge, etc. In an example, a blending parameter may be encoded in the frequency of the audio file, whereas in other examples an encoded blending parameter may be encoded in the RMS, the pitch or any other audio feature.

In the present specification, a "user interface" may comprise any device comprising means for interacting with a user such as a tactile screen, a microphone, a speaker, etc.

A "customized audio file" may be an audio file comprising a combination of one or more respective audio files associated to cartridges, wherein the customized audio file has been confirmed by a user to be sent to a fragrance dispensing device which dispenses a customized fragrance according to such customized audio file.

"Decoding the customized audio file" to obtain a set of customized blending parameters comprises splitting or decomposing the customized audio file thereby obtaining a receipt of the scents to be extracted and their respective proportions to create a customized fragrance composition.

Dispensing a customized fragrance composition based on the customized blending parameters comprises mixing one or more scents extracted from the cartridges of a dispensing device e.g. in a removable vessel.

In some examples, instead of having a (single) corresponding audio file associated to each cartridge each cartridge may comprise a plurality of associated audio files. Each audio file of such plurality of associated audio files may correspond to a different amount or proportion of the scent rather than having a single audio file which may be modified according to the amount to be released. In some examples, the plurality of associated audio files of a cartridge may also comprise some audio files corresponding to different music styles.

In some examples, receiving a customized audio file may comprise providing, via a user interface, a plurality of cartridge representations to a user, each cartridge representation corresponding to one of the plurality of cartridges. Then, the method may comprise selecting, via the user interface, a first cartridge representation and playing, upon selecting the first cartridge representation, the audio file associated to such first cartridge representation. The method may also comprise selecting, via the user interface, a second cartridge representation; combining the audio file associated to the first cartridge representation with the audio file associated to the second cartridge representation thereby obtaining a combined audio file and playing the combined audio file. Finally, method comprises, upon validation of the combined audio file by a user, generating a customized audio file comprising at least the combined audio file.

Combining audio files renders the customization of a fragrance easy and very user friendly. The method advantageously may save costs since unsatisfactory dispensed fragrances are avoided. The method also facilitates the creation of complex fragrances.

In some examples, the second cartridge representation may be iteratively selected by the user, until a validation of the combined audio file is received thereby enhancing the versatility and flexibility to customize a fragrance.

In some examples, the method may further comprise querying the user to select, via the user interface, a predetermined amount of a first cartridge to be extracted and modifying the corresponding audio file of the selected cartridge according to the predetermined amount to be released. The versatility and the preciseness may thus be further improved and/or enhanced.

In some examples, receiving, via a user interface, a customized audio file may comprise inputting a pre-existing customized audio file by the user may be advantageous since the creation of a customized fragrance will be done quickly and even in a single step, if the user is pleased by the audio file.

In a second aspect, the disclosure presents a computer program comprising program instructions for causing a computing system to perform a method according to any of the disclosed examples is provided.

In a third aspect, the disclosure presents a system for blending a customized fragrance is provided. The system comprises a user interface, configured to provide a plurality of cartridge representations, a control module configured to receive the customized audio file from the data input element and a fragrance dispensing device. Each of the plurality of cartridge representation is associated with a respective audio file of a plurality of audio files, and wherein the user interface further comprises a data input element for receiving a customized audio file. The fragrance dispensing device comprises a plurality of cartridges, each cartridge comprising one or more than one scent; a dispensing actuator configured to cause the release of a predetermined amount of scent from a selected cartridge, and a removable vessel for receiving the released predetermined amount of scent. The system is configured to perform a method according to any of the disclosed examples.

### BRIEF DESCRIPTION OF THE DRAWINGS

Particular embodiments of the present device will be described in the following by way of non-limiting examples, with reference to the appended drawings, in which:
Figure 1 is a schematic of a system for dispensing a customized fragrance according to an example;
Figure 2A and 2B schematically depict a user interface according to examples;
Figure 3 illustrates a flow chart of a method for dispensing a customized fragrance according to an example;
Figure 4 illustrates a flow chart of a method for receiving a customized audio file according to an example; and
Figure 5 illustrates a flow chart of a method for receiving a customized audio file according to an example.

### DETAILED DESCRIPTION

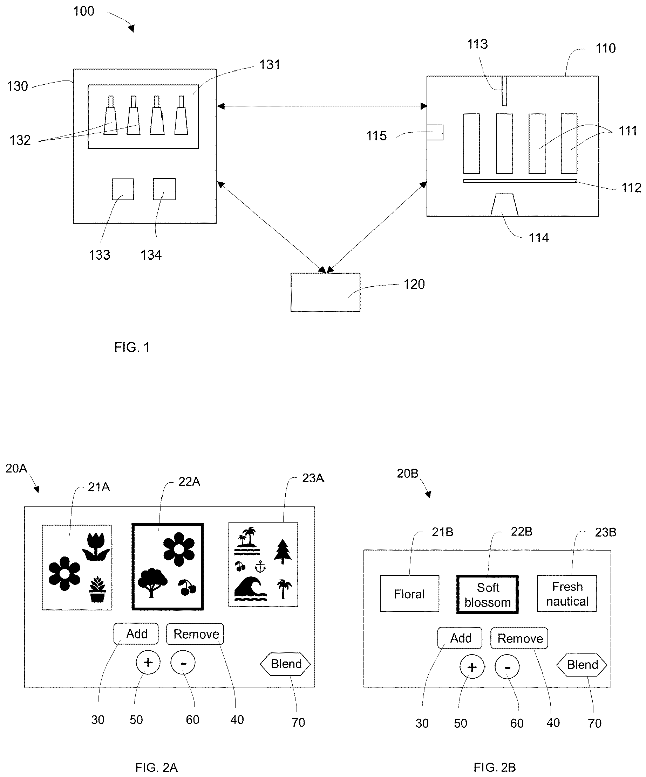

Figure 1 depicts a system 100 for blending customized fragrances. The system may comprise a fragrance dispensing device 110, a control module 120 and a user interface module 130.

The fragrance dispensing device 110 may comprise a plurality of cartridges 111, each cartridge comprising a predetermined amount, e.g. 150 ml, of one or more than one scent and/or essence. In cases wherein a cartridge comprises more than one scent, the amount of each scent (or note) may vary. Depending on the number of scents contained in each cartridge, there may be at least two different cartridge types: base cartridges comprising e.g. six scents, and chord cartridges comprising less scents than the base cartridge, e.g. three scents, which render the chord cartridges less complex than the base cartridges.

The cartridges 111 may comprise a releasing mechanism (not shown), e.g. a pump mechanism, configured to extract a predefined or a minimum amount of content from the cartridge, and also a releasing button for actuating such releasing mechanism. In some cases, the required amount to be extracted is greater than the minimum amount dispensed by the releasing mechanism. In such cases, the releasing mechanism may be further actuated, i.e. as many times as necessary, until the required amount is dispensed. In order to release the dispensed content, each cartridge may further comprise an output conduit.

Each cartridge, i.e. and thus the scent blend therein, may comprise a predefined corresponding audio file, i.e. an auditive representation, associated that may depend on the scent(s) and/or essence(s) contained on the cartridge. The corresponding audio file may comprise a plurality of musical notes or a simple melody which may be played by a single or a plurality of different musical instruments e.g. by a violin and/or a piano. For example, the audio file associated to a floral scent blend may comprise four musical notes played by a piano, and the audio file associated to the sandalwood scent may comprise a melody played by a drum.

In an example, each corresponding audio file may comprise at least two variations e.g. jazz and pop style, which may be switched upon selection e.g. via a selection element, of the user interface, such as a button. The variation of the audio file may comprise varying the style of the audio file according to user preferences, e.g. jazz style or pop style.

The corresponding audio files may be configured to be modified e.g. depending on the amount of the associated scent to be released. For instance, the intensity of the audio file may increase when the amount to be released is other than the minimum amount to be dispensed by the released mechanism. The corresponding audio files may be configured to be modified by changing their volume, pitch, attack, tone, length, range of frequencies, sound wavelength, intensity and/or frequency.

In some examples, instead of having a corresponding audio file associated to each cartridge, each cartridge may comprise a plurality of associated audio files. Each audio file of such plurality of associated audio files may correspond to a different amount or proportion of the scent rather than having a single audio file which may be modified according to the amount to be released. In some examples, the plurality of associated audio files of a cartridge may also comprise some audio files corresponding to different music styles.

The corresponding audio files may also be configured to be combined with at least another corresponding audio file thereby obtained a combined audio file. The combined audio file may be generated either by iteratively arranging or by superimposing at least two (different) corresponding the audio files.

Each corresponding audio file may comprise a set of encoded blending parameters containing information about e.g. the composition, the amount to be released, of a particular cartrige. In an example, blending parameters may be coded in the frequency of the audio file. The blending parameters may be modified upon modification e.g. of the proportion of the scent.

In an example, although a scent blend or cartridge may comprise a predetermined corresponding audio file associated, the user may the replace such predetermined corresponding audio may be another audio file.

The fragrance dispensing device may also comprise a rotatable carrousel 112 having a plurality of seats (not shown) for placing the plurality of cartridges. The rotatable carrousel 112 may rotate in order to iteratively arrange each selected cartridge in the actuating position i.e. a position in which the cartridges may be actuated to release a predetermined amount of the content.

The fragrance dispensing device 110 may also comprise a dispensing actuator 113 e.g. a vertically movable plunge, configured to actuate the cartridge arranged at the actuating position thereby causing the release of a predetermined amount of the content into a removable vessel 114.

In an example, each cartridge may comprise a read/write identification element such as a RIFD tag (not shown) which may comprise permanent data, i.e. data which may not need to be updated or re-written, such as the type of fragrance, and may also comprise non-permanent data, e.g. the remaining fragrance amount, which may be re-written in order to be updated. In such examples, the fragrance dispensing device 110 may comprise a read/write antenna 115, e.g. a RFID board, that may read the information of each identification element as the fragrance cartridges are rotated. The read/write antenna may also (re)write a read/write identification element arranged in each fragrance cartridge to update non-permanent data, e.g. the remaining fragrance volume.

As shown in Figure 1, the system 100 may comprise a user interface module 130 comprising a user interface 131 configured to provide a plurality of cartridge representations 132. The cartridge representations 132 provide information about the content of each cartridge in a user-friendly manner.

In an example, the cartridge representations may be visual representation (see Figure 2A) of the content of the corresponding cartridge. In case a cartridge comprises a plurality of scents, e.g. three scents, the cartridge representation may include a visual representation of each scent. In addition, the proportions of each of the plurality of scents may vary in a scent blend of a cartridge and thus, their visual representations may vary accordingly e.g. the size of the visual representation may be greater for scents having a greater proportion in the blend.

In another example, the cartridge representations may comprise or may be the name (see Figure 2B), e.g. "Floral", of the scent or the pre-mixed blend contained in the corresponding cartridge. In cases wherein a cartridge comprises more than one scent, the cartridge representation may be the name of the blend, e.g. "Floral", or may comprise the name of each scent of the blend e.g. "rose", "green citrus", etc. In addition, as the proportions of each of the plurality of scents may be different, the size of the representation may also vary accordingly e.g. the letter size may be greater for scents having a greater proportion in the blend.

In an example, the user interface 132 may be configured to darken e.g. the periphery of a selected cartridge upon selection (see element 22A of Figure 2A) in order to facilitate the user interaction.

Figure 2A and 2B illustrate two user interfaces 20A, 20B each displaying three selectable cartridge representations according to two alternative examples. Figure 2A depicts a user interface 20A wherein the cartridge representations 21A, 22A, 23A are visual representations of the content of each corresponding cartridge. Namely, a first and second cartridge representations 21A, 22A comprising three scents, and a third cartridge 23A comprising six scents. In the figure, second cartridge representation 22A is selected.

Besides, Figure 2B depicts an alternative example of a user interface 20B wherein the cartridge representations 21B, 22B, 23B are the names of the content of each corresponding cartridge. More specifically, the figure shows a first cartridge 21B represented by the term "Floral", a second cartridge 22B represented by the term "Soft blossom" and third cartridge representation 23B represented by the term "Fresh nautical". In the figure, the cartridge representation 22B is selected.

The user interface 131, 20A, 20B may also comprise a plurality of elements so as to facilitate the user interaction (see Figures 2A and 2B). The user interface may comprise an element 30 to confirm the selection of a selected cartridge representation, e.g. an "Add" button; and an element 40, e.g. a "Reject" button, to delete the selected cartridge representation. The rejection of a cartridge representation may be performed at any moment before the blending process is started. In an example, a cartridge representation may be rejected simply by not confirming. The user interface may further comprise an element 70, e.g. a "Blend" button, to start the blending process, i.e. to definitively confirm the selected cartridges.

The user interface 131, 20A, 20B may also comprise elements to regulate the amount of content to be released of a selected cartridge representation. In an example, the user interface may comprise an element 50 to increase the amount of content to be released e.g. a button comprising the "+" symbol, and an element 60 to reduce the amount to be released e.g. a button comprising the "-" symbol.

By providing different options to modify the selected and or the amount of the selected cartridge representation(s) the versatility and user friendliness may be enhanced.

In an example (not shown), the cartridge representations may be acoustic signals emitted by the user interface i.e. the user may reproduce the names of the scent blends in the cartridges and their proportion. In such examples, the user may orally: select, confirm, reject, modify the amount, start the blending process, etc. by e.g. speaking on a microphone.

In an example, the user interface 131 may be a tactile screen configured to display the cartridge representations. In another example, the user interface may be a system comprising a microphone 133 and a loudspeaker 134.

The user interfaces according to any of the disclosed examples may be used and/or included in many types of platforms. For example, the user interface may be configured to be implemented in personal and/or wearable devices e.g. smartphone, tablet, laptop, personal computer, etc. In another example, the user interface may be implemented e.g. as an app downloadable and executable at a corresponding personal and/or wearable device.

The user interface module 130 may further comprise a loudspeaker 134 for playing the audio files associated to the cartridges and the combined audio files and a microphone 133 for enabling a direct input of audio files e.g. when the user orally reproduces the name of the scent of a cartridge, in case wherein the cartridge representations are acoustic signals.

The control system 120 of the system of Figure 1 may be configured to receive the customized audio file from the user interface module 130. In addition, the control system 120 may also be configured to decode the customized audio file and obtain a set of customized blending parameters, and to send such customized blending parameters to the dispensing device 110 which may be configured to extract the one or more scents from the cartridges according to provided blending parameters thereby dispensing a customized fragrance composition.

In an example (not shown), the control system may be part of the user interface module.

The system device may comprise a communication system for connecting the user interface module, the control module and the fragrance dispensing device. The communication system may be configured to receive a customized audio file and to send the customized blending parameters. In an example, the communication system may be a wireless communication system such as Bluetooth, Wi-Fi, etc.

The systems according to Figure 1 are a combination of electronic and computing means, the computing means may be a set of instructions, e.g. a computer program, and the electronic means may be any electronic circuit capable of implementing corresponding method steps of the proposed fragrance dispensing method(s).

The computer program(s) may be embodied on a storage medium, e.g. a CD-ROM, a DVD, a USB drive, a computer memory or a read-only memory; or carried on a carrier signal e.g. on an electrical or optical carrier signal.

The computer program(s) may be in the form of source code, object code, a code intermediate source and object code such as in partially compiled form, or in any other form suitable for use in implementing the fragrance dispensing method(s) according to any of the disclosed examples. The carrier may be any entity or device capable of carrying the computer program(s).

For example, the carrier may comprise a storage medium, such as a ROM, e.g. a CD ROM or a semiconductor ROM; or a magnetic recording medium, e.g. a hard disk. Further, the carrier may be a transmissible carrier such as an electrical or optical signal, which may be conveyed via electrical or optical cable or by radio or other means.

When the computer program(s) is/are embodied in a signal that may be conveyed directly by a cable or other device or means, the carrier may be constituted by such cable or other device or means. Alternatively, the carrier may be an integrated circuit in which the computer program(s) is/are embedded, the integrated circuit being adapted for performing, or for use in the performance of, the proposed methods.

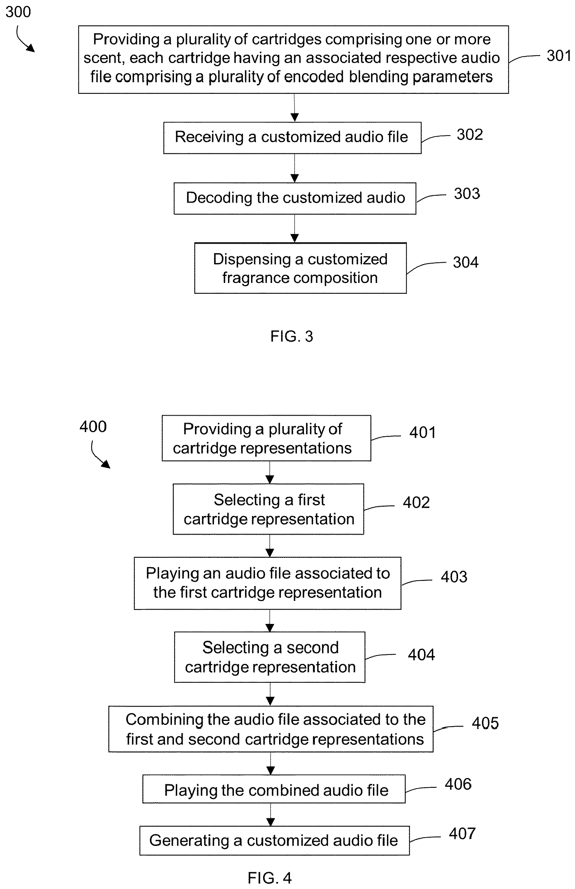

Figure 3 is a flow chart schematically illustrating a customized fragrance dispensing method 300 according to an example. Such fragrance dispensing method is performable by/through systems similar to the one described with reference to Figure 1.

Firstly, a plurality of cartridges comprising one or more scents may, in block 301, be provided. Each of the provided cartridges may be associated with a respective audio file, wherein each audio file comprises a plurality of encoded blending parameters.

A customized audio file may, in block 302, be received via a user interface. The customized audio file may be received by way of any of the examples given further below.

The customized audio file may then, in block 303, be decoded to obtain a set of customized blending parameters. The customized blending parameter may comprise the scent blends and the amount of each blend required to dispense a customized blend e.g. by a fragrance dispensing device.

In an example, decoding the customized audio file comprises using a decoding algorithm. The decoded customized blending parameters having the information about the scents and their amount to be released may be then send e.g. to a fragrance dispensing device.

Then, a customized fragrance composition based on the customized blending parameters may, in block 304, be dispensed e.g. by extracting the required amount of the scents according to such blending parameters.

In an example, after dispensing the customized fragrance composition, the user may be queried to confirm the customized audio file corresponding or associated to the dispensed fragrance. The user may substitute the audio file by another preferred audio file.

The customized audio file may be received by way of any of the following examples. In an example represented by method 400, receiving the customized audio file may comprise actively selecting a plurality of cartridge representations which upon selection may progressively generate a customized audio file. In a further example represented by method 500 receiving the customized audio file may comprise directly selecting the customized audio file e.g. a pre-existing audio file from a list.

Figure 4 depicts a flow chart of a method 400 for receiving a customized according to an example.

Firstly, the method 400 may comprise providing, in block 401, a plurality of cartridge representations to a user according to any of the disclosed examples. The cartridge representations may be provided via a user interface, e.g. a tactile screen, and each representation may correspond to one of a plurality of cartridges.

In an example, the provided plurality of cartridge representations may correspond to the cartridges that are available, i.e. that are arranged, in a fragrance dispensing device. In another example, the provided plurality of cartridge representations may correspond to a list of commercially available cartridges, and a warning may be displayed in cases where a provided cartridge is not available at the fragrance dispensing device.

The user may, in block 402, select a first cartridge representation. In examples wherein the cartridge representations are visual representations, the user may interact with the user interface, e.g. by touching the tactile screen, in order to select a first cartridge representation. In examples wherein the cartridge representations are auditive representations, the user may select a first cartridge by inputting in a microphone the name of the cartridge as an acoustic signal.

Upon selection of the first cartridge representation, the respective audio file associated to such first cartridge representation may, in block 403, be played via a loudspeaker. The audio file may be continuously repeated e.g. until a confirmation or a further cartridge is selected.

In the event the user is pleased by the played audio file, she/he may confirm the selection of such first cartridge e.g. by clicking a displayed "Add" button. On the contrary, if the user is not pleased by the audio file associated to the selected first cartridge, i.e. with the played audio file, the user may select a further (first) cartridge representation(s) until a confirmation is received.

In an example, the user may select or may be queried to selected, via the user interface, a predetermined amount of the first cartridge to be released. The predetermined amount may be selected before or after confirming the selection of such first cartridge.

In addition, and depending on the selected amount, the corresponding audio file of the selected first cartridge may be modified accordingly. The audio file may be modified for instance by changing the volume, pitch, attack, tone, length, range of frequencies, sound wavelength, intensity and/or frequency. The modified audio file may then be played.

The amount to be released may be repeatedly varied, i.e. the proportion may be increased or decreased, until the user is satisfied by the played audio file. After each variation, the audio file may be modified and played in order to check that the modified audio file pleased the user.

After receiving the confirmation of the first cartridge representation, e.g. by pressing and "Add" button, the user may, in block 404, select a second cartridge representation from the plurality of provided, i.e. displayed, cartridge representations.

In an example, the plurality of cartridges provided for successively selected cartridges i.e. for second and subsequent cartridge representations, may depend on the content of such first cartridge i.e. the selectable options may be limited to cartridge representations that may lead to a technically correct fragrance blend. Additionally or alternatively, the provided cartridges may correspond to profiling and/or machine learning technics which may provide cartridge representations suitable to be enjoyed by the user.

Upon selection of a second cartridge representation, the audio files of first and second cartridge representations may, in block 405, be combined thereby generating a combined audio file. The combined audio file be may generated by subsequently adding the corresponding audio files of first and second cartridge representations; or by superimposing the corresponding audio files. In an example, the user may select the way in which the combined audio file is generated e.g. via a button.

The combined audio file may, in block 406, be played. In the event the user is pleased with the combined audio file, she/he may confirm the selection of the second cartridge representation, e.g. by clicking a displayed "Add" button. As a result, a customized audio file may, in block 407, be generated.

The process may be repeated until a plurality, i.e. at least two, cartridges are selected. In the event more than two cartridge representations are selected and confirmed, the customized audio file may be generated when the last cartridge representation is confirmed.

In an example, before or after confirming the selection of the second cartridge, the user may select or may be queried to select a, via the user interface, a predetermined amount of the second cartridge representation. Besides, and depending on the selected amount, the corresponding audio file of the selected second cartridge may be modified accordingly thereby also modifying the combined audio.

The user may repeatedly vary the selected amount, i.e. may increase or to decrease, the proportion, any selected cartridge representation before or after confirming each cartridge representation. In an example, the user may vary the selected amount of each selected cartridge representation at any time before dispensing the fragrance. That is, the variation of the proportion of any of the selected cartridge representation would result in the variation of the combined audio file. The audio file may be played after each variation in order to check whether the modified combined audio file pleases the user.

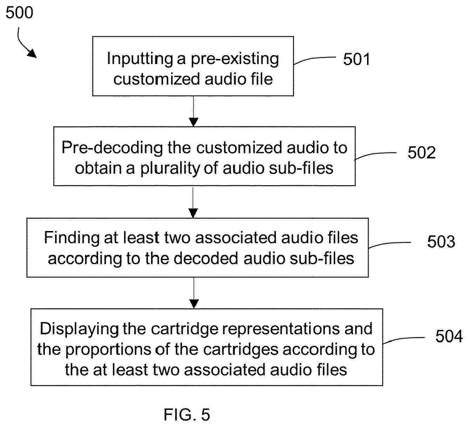

Figure 5 depicts a flow chart of a method 500 for receiving a customized according to an alternative example.

A customized pre-existing customized audio file may, in block 501, be input via the user interface, by the user e.g. through a tactile screen. The user interface may comprise a list, e.g. recorded in a memory, comprising a plurality of pre-existing audio files registered therein. Such list may be configured to download a selected audio file from the plurality of favourite audio files recorded and also to upload e.g. from the internet, further audio files. The list may be e.g. a Spotify®, Amazon Music, etc. music play list. The pre-existing audio files may be complex audio files such as commercial songs, melodies created or recorded by the user, etc.

The customized audio file may, in block 502, be pre-decoded to obtain a plurality of audio sub-files which treatment may be more efficient, accurate and/or faster to process than the pre-existing customized audio file. The pre-decoding process may be carried out by an algorithm which splits the customized audio file into individual audio tracks or sub-files, which may also include finding, in block 503, at least two corresponding audio files, i.e. and thus the cartridges to which they are associated, according to such audio sub-files.

That is, the algorithm may compare the decoded audio sub-files and the audio files associated to all available cartridges, e.g. the cartridges available at the fragrance dispensing device or all the commercially available cartridges, and find at least two corresponding audio files which are more similar, i.e. have more properties in common e.g. similar frequency or tone; to the decoded audio sub-files. Moreover, the algorithm may also compare the audio sub-files with variations of the corresponding audio file in order to specify the proportion of each scent blend i.e. the amount to be extracted from each cartridge.

The combination of the at least two corresponding audio files or of their variations may be similar to the input pre-existing customized audio file.

The cartridge representations and the proportions of the least two cartridges according to the at least two corresponding audio files may, in block 504, be provided e.g. displayed via the user interface. The user may then confirm or reject the provided cartridges representations and/or the proportion of each cartridge representation. In an example, the user may also modify, i.e. increase or decrease, the proportion of each cartridge representation.

In an example, the dispensing method 500 may comprise, after receiving a pre-existing audio file, directly proceed to blend the fragrance according to the customized blended parameters decoded, that is, without permitting any change or modification.

Although only a number of particular embodiments and examples have been disclosed herein, it will be understood by those skilled in the art that other alternative embodiments and/or uses of the disclosed innovation and obvious modifications and equivalents thereof are possible. Furthermore, the present disclosure covers all possible combinations of the particular embodiments described. The scope of the present disclosure should not be limited by particular embodiments, but should be determined only by a fair reading of the claims that follow.

## Claims

1. A method for dispensing a customized fragrance by a fragrance dispensing apparatus, the method comprising:
providing a plurality of cartridges comprising one or more scents, wherein each cartridge is associated with a respective audio file of a plurality of audio files, wherein each audio file comprises a plurality of encoded blending parameters;
receiving, via a user interface, a customized audio file;
decoding the customized audio file to obtain a set of customized blending parameters; and
dispensing a customized fragrance composition based on the customized blending parameters.

2. The method according to claim 1, wherein receiving a customized audio file comprises:
providing, via a user interface, a plurality of cartridge representations to a user, each cartridge representation corresponding to one of the plurality of cartridges;
selecting, via the user interface, a first cartridge representation;
playing, upon selecting the first cartridge representation, the audio file associated to such first cartridge representation;
selecting, via the user interface, a second cartridge representation;
combining the audio file associated to the first cartridge representation with the audio file associated to the second cartridge representation thereby obtaining a combined audio file;
playing the combined audio file; and
upon validation of the combined audio file by a user, generating a customized audio file comprising at least the combined audio file.

3. The method according to claim 2, wherein the second cartridge representation is iteratively selected by the user, until a validation of the combined audio file is received.

4. The method according to claim 2 or 3, wherein, in response to receiving a rejection of the combined audio file, the method further comprises:
rejecting the second cartridge representation, and
selecting a further second cartridge representation.

5. The method according to any of claims 2-4, wherein providing the plurality of cartridge representations to the user comprises displaying a name or a visual representation of each cartridge and/or of its content.

6. The method according to any of claims 2-5, wherein selecting the first cartridge comprises a tactile gesture via the user interface.

7. The method according to any of claims 2-5, wherein selecting the first cartridge comprises using an acoustic signal.

8. The method according to any of claims 2-7, further comprising:
querying the user to select, via the user interface, a predetermined amount of a first cartridge to be released; and
modifying the corresponding audio file of the selected cartridge according to the predetermined amount to be released.

9. The method according to claim 8, wherein modifying the corresponding audio of the selected cartridge comprises changing the volume, pitch, attack, tone, length, range of frequencies, sound wavelength, intensity and/or frequency of the respective audio file.

10. The method according to claim 1, wherein receiving, via a user interface, a customized audio file comprises inputting a pre-existing customized audio file by the user.

11. The method according to claim 10, further comprises:
pre-decoding the customized audio file to obtain a plurality of audio sub-files;
finding at least two respective audio files of the plurality of audio files according to the audio sub-files, and
displaying the cartridge representations and the proportions of the cartridges of the at least two respective audio files.

12. A computer program comprising program instructions for causing a computing system to perform a method according to any of claims 1 to 11.

13. A system for blending a customized fragrance, the system comprising:
a user interface configured to provide a plurality of cartridge representations, each of the plurality of cartridge representation is associated with a respective audio file of a plurality of audio files, and wherein the user interface is further configured to receive a customized audio file;
a control module configured to receive the customized audio file from the user interface; and
a fragrance dispensing device comprising:
a plurality of cartridges, each cartridge comprising one or more than one scent,
a dispensing actuator configured to cause the release of a predetermined amount of scent from a selected cartridge, and
a removable vessel for receiving the released predetermined amount of scent; and wherein
the system is configured to perform the method of any of claims 1 - 11.

14. Method for dispensing a customized fragrance, comprising:
providing a plurality of cartridges comprising one or more scents, wherein each cartridge is associated with a respective audio file of a plurality of audio files, wherein each audio file comprises plurality of encoded blending parameters;
extracting the one or more scents from the cartridges according to a set of customized blending parameters; which customized blending parameters are received from a user interface; and
dispensing a customized fragrance composition based on the blending parameters.

15. Method for requesting a customized fragrance, comprising
providing a plurality of cartridges comprising one or more scents, wherein each cartridge is associated with a respective audio file of a plurality of audio files, wherein each audio file comprises plurality of encoded blending parameters;
receiving, via a user interface, a customized audio file; and
decoding the customized audio file to obtain a set of customized blending parameters,
requesting the customized fragrance composition by sending the customized blending parameters to a fragrance dispensing device.
